# NEW EUROPEAN PATENT SPECIFICATION

(11) **EP 0 381 445 B2**
(45) Date of publication and mention of the opposition decision: **28.01.1998**
(45) Mention of the grant of the patent: 04.05.1994
(21) Application number: 90300952.0
(22) Date of filing: 30.01.1990
(51) Int. Cl.: A61K 6/00

(54) **Dental composition for hypersensitive teeth**
Dentalzusammensetzung für überempfindliche Zähne
Composition dentaire pour les dents hypersensibles

(30) Priority: 31.01.1989 US 304091
(43) Date of publication of application: 08.08.1990
(73) Proprietor: YISSUM RESEARCH DEVELOPMENT COMPANY OF THE HEBREW UNIVERSITY OF JERUSALEM, Jerusalem 91042 (IL)
(72) Inventor: Friedman, Michael, Jerusalem 93833 (IL)
(74) Representative: Sheard, Andrew Gregory

(56) References cited:
- EP-A- 264 660
- EP-A- 0 128 655
- EP-A- 0 165 454
- EP-A- 0 223 245
- EP-A- 0 241 295
- GB-A- 990 957
- US-A- 4 057 621
- US-A- 4 415 549
- US-A- 4 631 185
- US-B- 3 863 006
- Römpp (Band 4/1991), pp. 2941-2942
- J. Biol. Buccale, vol. 15,, pp. 71-82, 1987 "Fuoride varnisches - A review", H. de Bruyn et al
- H.P. Fiedler Lexikon der Hilfsstoffe für Pharmazie, Kosmetik und angrenzende Gebiete, 1981, page 527
- Rote Liste 1990, 53001
- Vivadent information 10/91
- Biomaterials 1984, vol. 5, "Bioadhesive intraoral release systems : design, testing and analysis", R. Gurny et al

## Description

This invention is directed to a dental composition for preventing hypersensitivity in teeth, its use in medicine and in the preparation of an agent for the treatment of hypersensitive teeth.

EP-A-0241295 discloses pharmaceutical compositions for use in desensitising hypersensitive dentin that contain N-methacryloyl aminosalicylic acid (NMSA) in a liquid carrier, the compound having an unsaturated group and a polymerisation initiator. Such compositions need to be stored until use in a cold or dark place in order to prevent the occurence of copolymerisation.

US-B-3963006 refers to alkali metal nitrate compositions for use in treating tooth hypersensitivity that can function as a toothpaste and contains water.

US-A-4631185 discloses aqueous solutions of potassium salts for reducing sensory nerve activity in hypersensitive teeth.

EP-A-0165454 relates to water based suspensions containing antihypersensitivity agents that are rubbed or mechanically pressed against teeth.

Dental hypersensitivity, especially that arising from dentin and cementum hypersensitivity, is a frequently encountered problem in dentistry and a very troublesome clinical complaint. Hypersensitivity may occur wherever the dentin or cementum of a tooth is exposed by attrition or abrasion, or when the tooth's fine root surface is exposed by periodontal disease. In about 12% of erupted teeth, there is a developmental lack of protective covering of cementum at the cementoenamel junction. As a result, when the exposed dentin is subjected to mechanical, thermal, chemical or osmotic stimuli, the sensory nerves of the teeth become excited and a very painful response results. For example, people with hypersensitive teeth find it very painful to orally ingest certain forms of nourishment, such as liquids or foods that are hot or cold, sweet, hypertonic or contain citric acid. Everyday stimuli such as brushing the teeth may also be painful.

Many attempts have been made to control hypersensitivity of the teeth. For example, U.S. Patent No. 3,863,006 (Hodosh, M.) describes the use of potassium, lithium or sodium nitrate; U.S. Patent No. 4,751,072 and U.S. Patent No. 4,631,185 (both to Kim, S.) describe the use of potassium bicarbonate and potassium chloride; U.S. Patent No. 4,710,372 and U.S. Patent No. 4,634,589 (both to Scheller, H.U.) describe the use of hydroxyapatite or fluorapatite; U.S. Patent No. 4,057,621 Pashley, D.H., et al.) describes the use of an alkali metal or ammonium oxalate; U.S. Patent No. 4,415,549 (Shah, N.B.) describes the use of strontium EDTA, fluoride and ammonium glycyrrhizzinate; and, GB990957 (Rosenthal, M.W.) describes the use of strontium for the control of hypersensitivity. The use of strontium ions to treat hypersensitivity was also disclosed in U.S. Patent Nos. 3,122,483, 3,988,434 and 4,224,310.

However, although clinically the most effective for reducing tooth hypersensitivity, the use of strontium salts for the treatment of hypersensitivity is disliked by patients due to the tendency of strontium salts to leave an unacceptably salty taste or metallic taste in the mouth, even when used in a toothpaste form. Another major disadvantage of strontium dentifrice is the long period of time of application which is required to achieve the clinical effect.

A topical, sustained-release form of an agent capable of controlling dental hypersensitivity could help prevent undesirable taste side effects and still treat the hypersensitive condition., Such a dosage form would be able to release the agent controlling the hypersensitivity at a lower therapeutic level over a long period of time, for example, for weeks. Sustained localized release of the hypersensitivity agent, targeted directly to the hypersensitive site, would also solve the problem of the prolonged time and application currently required to obtain clinical effectiveness with strontium.

Sustained release of an agent to treat a dental disease, peridontal disease, has been reported to be achieved by embedding chlorhexidine in an ethyl cellulose polymer to form a varnish (Friedman, M., et al., J. Perio. Res. 17:323-328 (1982); Friedman, M., et al., IADR Prog. and Abstr. 59:No. 905 (1980); Soskolne, W.A., et al., J. Perio. Res. 18:330-336 (1983)). This dosage form was also used in the treatment of plaque prevention in patients wearing orthodontic appliances (Friedman, M., et al., J. Dent. Res. 64:1319-1321 (1985)). However, this treatment, termed a varnish because it is applied to the surface of the teeth or tissues, was not deemed useful for the long-term prevention of the dental condition. Thus a need exists for the identification of a varnish composition capable of supplying a dental agent in an efficacious sustained-release, long term dosage form.

Wahmi (U.S. Patent No. 4,374,824) discloses dentifrices for cleaning and preserving teeth. Disclosed were compositions comprising ginger, magnesium silicate, sodium chloride, catechu, alum, seed and shell of sweet almond, pyrethrum, gum mastic, and tobacco. It was reported that gum mastic was added to the composition to assist in the prevention of tooth decay. The disclosed compositions were intended to be in the form of toothpaste or tooth powders. The Wahmi patent does not disclose the possible long-term anti-hypersensitivity effect of the compositions; further, application of the disclosed compositions two to three times per day is required for antiplaque activity.

Mastic has been used previously for other dental purposes. U.S. Patent No. 4,668,188 (Wolfenson, G.B.) discloses the use of a curable mastic in the production of an oral impression tray for making impressions of teeth and jaw structures. Mastics have been used in the production of dental molds (U.S. Patent No. 4,500,288, VonWeissenfluh, H.) and as an adhesive to secure dental articulators (U.S. Patent Nos. 4,548,581 and 4,382,787, Hoffman, R.E.). U.S. Patent Nos. 4,532,126 and 4,428,927 (Ebert, W.R., et al.) disclose chewable, filled, one-piece soft elastic gelatin capsules, made chewable by a masticatory substance, such as a synthetic mastic.

U.S. Patent No. 4,459,277 (Kosti, C.M.) relates to novel plaque compositions for use in evaluating oral hygiene practices. In brief, the patent discloses a water-insoluble, water-immiscible dye emulsified in fine droplets or rupturable capsules. The patent discloses the use of mastic resin as well as alginates, and other gums as an insoluble media for dye dispersion. In particular, sodium carboxymethylcellulose is disclosed. Also disclosed is the possibility of incorporating antibacterial agents such as stannous fluoride into the compositions. Significantly, the Kosti patent is concerned with diagnostic rather than therapeutic applications. The patent fails to suggest compositions exhibiting long-term preventive activity for hypersensitive teeth.

The background art fails to identify any compositions of matter comprising an effective anti-hypersensitivity agent together with a long term sustained release carrier capable of providing efficacious levels of the anti-hypersensitivity agent, either alone or in combination with an adhesive polymer such as a mastic and a plasticizer such as polyethylene glycol, for use as a hypersensitivity preventative by humans and other animals, under conditions in which the anti-hypersensitivity agents have no undesirable side effects such as changes in taste sensations.

The present invention seeks to provide an anti-hypersensitivity composition capable of delivering efficacious levels of an agent effective against those dental conditions responsible for hypersensitivity, the composition being such that the active anti-hypersensitivity agent may be released in a sustained, long-term fashion, without a salty or metallic taste, and such that the hypersensitivity composition may have the property of long-term adhesion to the teeth so that the hypersensitivity composition may remain plastic during the entire period of application. The hypersensitivity composition of the present invention can use materials already approved by the F.D.A..

Thus according to a first aspect of the invention there is provided a sustained release dental anti-hypersensitivity varnish composition comprising:
(a) an anti-hypersensitivity agent; and
(b) a sustained release hydrophobic polymer dissolved in a pharmaceutically acceptable solvent;
   the composition being adapted to dry to form a film on evaporation of the solvent, which film adheres to a surface of a tooth in a manner which resists removal under normal conditions such as eating or brushing of teeth, and which allows sustained release of the hypersensitivity agent so that a hypersensitive condition can be treated.

A second aspect of the present invention relates to the sustained release dental anti-hypersensitivity varnish composition of the first aspect for use in human or veterinary medicine.

According to a third aspect of the present invention there is provided the use of a composition comprising:
(a) an anti-hypersensitivity agent; and
(b) a sustained release hydrophobic polymer dissolved in a pharmaceutically acceptable solvent;
   in the preparation of the sustained release dental anti-hypersensitivity varnish composition according to the first aspect of the present invention.

A fourth aspect of the present invention relates to a process for the preparation of the sustained-release hypersensitivity preventative varnish composition according to the first aspect of the present invention, the process comprising admixing:
(a) an anti-hypersensitivity agent;
(b) a sustained release hydrophobic polymer; and a pharmaceutically acceptable evaporable solvent to dissolve the polymer.

In the following description preferred features and characteristics of one aspect of the present invention are applicable to another aspect *mutatis mutandis*.

The composition in the present invention is useful for preventing dental hypersensitivity, by applying the composition to teeth or gingival tissues, for example by brush or by spray, to the teeth of humans or (eg. domesticated) animals.

Thus the present invention encompasses a composition comprising a metal salt or other hypersensitivity agent (that is capable of suppressing dental hypersensitivity) embedded in a sustained release carrier composed of a cellulose or hydrophobic polymer, in a pharmaceutically acceptable solvent, optionally containing a plasticizer such as polyethylene glycol and/or an adhesive polymer such as gum mastic.

The invention encompasses oral compositions that may provide sustained, efficient, inexpensive, anti-hypersensitivity activity without deleterious or undesirable side effects, and methods for using such compositions.

By "sustained-release" is meant the continuous release of an active substance at efficacious levels for a prolonged period of time, such as 2-4 weeks or longer. The release of the active substance may be constant or pulsed, as long as efficacious levels of the active substance are provided to the surrounding milieu for the desired period of time.

By an "efficacious level" is meant a level or concentration of a drug or other active agent which is high enough to be effective in treating the condition the drug was designed to treat.

By "oral varnish" is meant a composition which is topically applied to a surface such as a tooth, and which dries as a film adhering to that surface, in a manner which resists removal under normal conditions, such as eating or brushing teeth.

The compilation of the components of the aforementioned oral composition is based upon the specific properties of each of the individual components, wherein each component of the combination increases the anti-hypersensitivity effectiveness of other members of the combination.

The oral composition of the invention may assist in the prevention of dental hypersensitivity. A variety of anti-hypersensitivity agents are suitable for the present invention. Preferred is the use of strontium salts. Other anti-hypersensitivity agents useful in the composition of the invention include alkali metal salts, such as nitrates, halides and bicarbonates, for example potassium, lithium or sodium nitrate, potassium bicarbonate, potassium chloride; as well as apatites such as hydroxyapatite, fluorapatite; also ammonium compounds such as ammonium oxalate and ammonium glycyrrhizzinate; and fluoride compounds, eg. fluoride chelates such as EDTA with fluoride.

Preferably the anti-hypersensitivity agent is released to the hypersensitive site in a long-term sustained release manner so as to reduce the required frequency of use. This kind of release may be accomplished by embedding the anti-hypersensitivity agent in a cellulosic or hydrophobic acrylic polymer to form a varnish for administration to the oral cavity. The use of these polymers may have the additional advantage of minimizing side effects of the hypersensitivity agent. Preferred are the insoluble and inexpensive polymers: hydrophobic type (polyethylene (which is particularly preferred), polymethacrylate, polyamide-nylon, poly(ethylene-vinyl acetate) cellulose nitrate, silicones and others). A preferred cellulosic polymer is ethyl cellulose.

In a preferred embodiment, the oral composition with the highly desirable characteristics mentioned above comprises an anti-hypersensitivity compound such as strontium, preferably a strontium halide, eg. chloride, embedded in a sustained release hydrophobic (eg. cellulosic) polymer such as ethyl cellulose, in a pharmaceutically effective solvent. For example, strontium (1-5 parts) and ethyl cellulose (5-9 parts) may be dissolved in ethanol (80-120 parts) for the preparation of sustained release delivery systems. The efficacy of such preparations demonstrates that the anti-hypersensitivity agent may be efficiently released from the varnish at efficacious levels for a period of at least four weeks. In another embodiment, combinations of strontium salts with another anti-hypersensitive agent are used.

For application to buccal and lingual surfaces of teeth, an alcoholic, eg. ethanolic, solution of the antisensitivity agent and cellulosic or hydrophobic polymer (containing up to 4% of the anti-hypersensitivity agent as used in the varnish) are applied with a soft brush or with a spray. The dry film is formed in situ, after application of the varnish to the tooth surface and evaporation of the solvent. Mouthwash forms are not suitable because of inefficient application of the composition to affected areas. Preferably, a film of 10-160 µm thick dries on the surface of the tooth.

Those skilled in the art of oral medicine will, without undue experimentation, be able to produce ranges of concentrations of other appropriate antisensitivity agents and sustained release polymers.

The oral compositions for hypersensitivity treatment and prevention may also comprise additional desirable components. For example, the adhesiveness of the oral composition may be improved by the incorporation within the composition of an adhesive polymer such as gums, eg. gum mastic in an amount of from 1-20% by weight, of the gum mastic. Other suitable mastics are disclosed in U.S. Patent No. 4,315,779 to Heyd, D., et al., and U.S. Patent No. 4,374,844 to Wahmi, H.V.R., et al.

The composition may include demulcents/humectants (i.e., plasticizers) such as polyethylene glycol (eg. 400-to-4000), glycerol, sorbitol, or mineral oil, eg. in concentrations of about 1% by weight. Other humectants, detergents, or surface-active agents will be known to those skilled in the formulation of oral compositions.

Thus, in a preferred composition, the oral composition of the invention comprises strontium, ethyl cellulose polymer, an adhesive, a plasticizer, and solvent (such as alcohol, eg. ethanol). In highly preferred compositions, gum mastic is also present. Flavourings and coloring agents may also be present as required.

A preferred composition suitably comprises:
(a) an anti-hypersensitivity agent, eg. strontium chloride, in an amount of from 2 to 3% w/v;
(b) a hydrophobic polymer, such as ethyl cellulose, in an amount of from 5 to 6% w/v;
(c) advantageously a plasticizer such as polyethylene glycol, in an amount of from 0.5 to 1.0% w/v;
(d) a solvent, preferably an alcohol such as ethanol.
(Amounts in % w/v are based on the solvent.)

The invention will now be described by way of example with reference to the following Examples, which are provided by way of illustration and are not to be construed as limiting on the present invention.

### Example 1

### Varnish Preparation

The following formulations (Table 1) were all prepared by the same general procedure as follows: ethyl cellulose and polyethylene glycol polymers were dissolved in the suitable solvent. After complete dissolution of the polymers, the additional components of the varnish were added.
Ethyl cellulose - EC
Polyethylene glycol - PEG
Strontium chloride - STR

**TABLE 1**

| MATERIAL/FORMULATION | | | | | | | | | |
|---|---|---|---|---|---|---|---|---|---|
| | I | II | III | IV | V | VI | VII | VIII | IX |
| STR (G) | 1.0 | 2.0 | 3.0 | 3.0 | 3.0 | 4.0 | 3.0 | 3.0 | 3.0 |
| EC-NF100 (G) | 8.5 | 7.0 | 6.0 | 6.0 | 6.0 | 5.0 | 6.0 | 6.0 | 6.0 |
| PEG 400 (G) | 0.5 | 1.0 | 1.0 | 1.0 | 1.0 | 1.0 | --- | --- | --- |
| PEG 4000 (G) | --- | --- | --- | --- | --- | --- | 1.0 | --- | --- |
| GLYCEROL (G) | --- | --- | --- | --- | --- | --- | --- | 1.0 | --- |
| MINERAL OIL (G) | --- | --- | --- | --- | --- | --- | --- | --- | 1.0 |
| ETHANOL (C''C) | 100 | 100 | 100 | 80 | 120 | 100 | 100 | 100 | 100 |

The best formulation was No. IV. The concentration of strontium in No. IV is lower than that currently used in toothpaste and dental solutions which contain 10% strontium.

### The Effect of Local Application of Sustained Release Varnish Containing Strontium on Hypersensitivity of Teeth

The effects of local application of a sustained-release delivery system of strontium on hypersensitive teeth is shown in Table 2.

The study includes nine patients, among whom 55 teeth were treated. After treatment with the strontium varnish formulation No. IV from Table 1, the teeth were tested again after 7 and 30 days. Sensitivity was rated on a scale of 0-5, with five being the highest sensitivity. None of the patients complained of a metallic or salty taste.

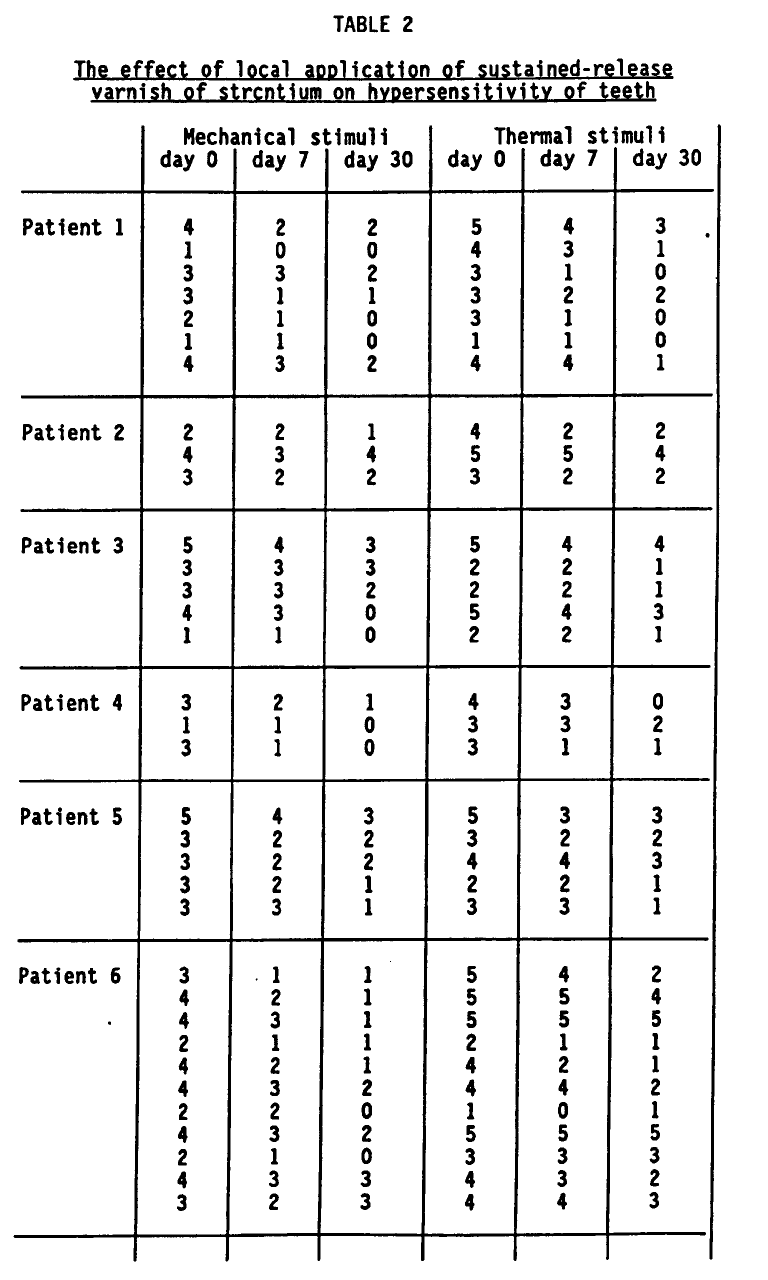

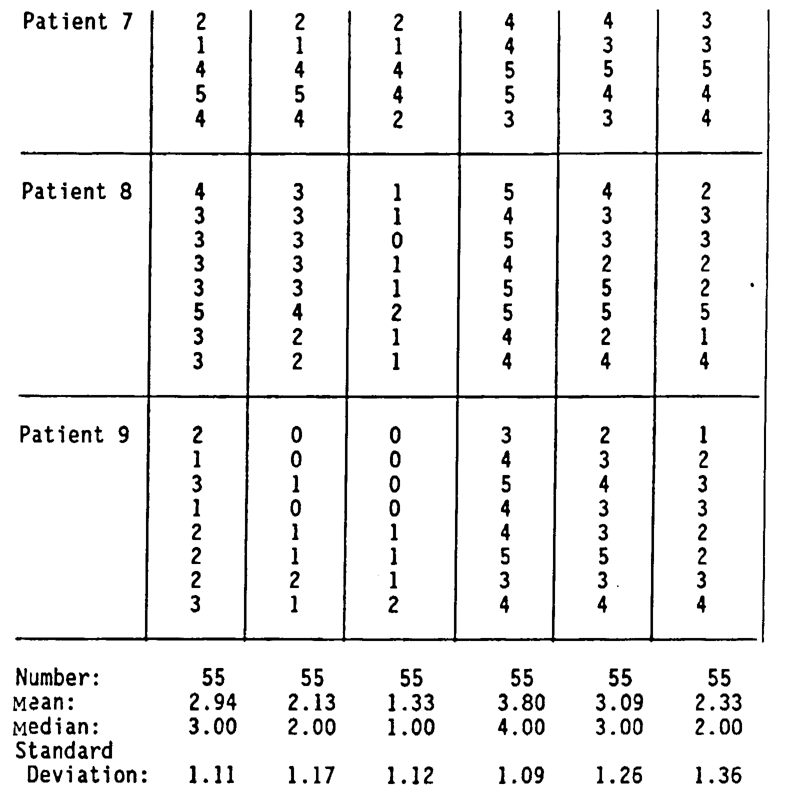

### Statistical Evaluation:

### Mechanical stimuli

- Day 7 -: significantly different than day 0 (PL10⁻⁶, pair t-test, two-talled) - tailed.
- Day 30 -: significantly different than day 0 (PL10⁻⁶, pair t-test, two-talled) - tailed.

### Thermal stimuli

- Day 7 -: significantly different than day 0 (PL10⁻⁶, pair t-test, two-talled) - tailed.
- Day 30 -: significantly different than day 0 (PL10⁻⁶, pair t-test, two-talled) - tailed.

The results in Table 2 show that most of the teeth varnished with a strontium-containing composition of the invention were no longer hypersensitive or showed significantly less hypersensitivity for as long as 30 days after application of the varnish.

## Claims (Claims for the following Contracting State(s): AT, BE, CH, DE, DK, FR, GB, IT, LI, LU, NL, SE)

1. A sustained-release dental anti-hypersensitivity varnish composition comprising:
(a) an anti-hypersensitivity agent; and
(b) a sustained release hydrophobic polymer dissolved in a pharmaceutically acceptable solvent;
the composition being adapted to dry to form a film on evaporation of the solvent, which film adheres to a surface of a tooth in a manner which resists removal under normal conditions such as eating or brushing of teeth, and which allows sustained release of the hypersensitivity agent so that a hypersensitive condition can be treated.

2. A sustained-release dental anti-hypersensitivity varnish composition comprising:
(a) an anti-hypersensitivity agent; and
(b) a sustained release hydrophobic polymer dissolved in a pharmaceutically acceptable solvent;
the composition being adapted to dry to form a film on evaporation of the solvent, which film adheres to a surface of a tooth in a manner which resists removal under normal conditions such as eating or brushing of teeth, and which allows sustained release of the hypersensitivity agent so that a hypersensitive condition can be treated;
for use in medicine.

3. A composition as claimed in claim 1 or 2 wherein the anti-hypersensitivity agent is a strontium salt, potassium, lithium or sodium nitrate, potassium bicarbonate, potassium chloride, hydroxyapatite, fluorapatite, ammonium oxalate, EDTA with fluoride, fluoride, or ammonium glycyrrhizzinate.

4. A composition as claimed in any of claims 1 to 3 wherein the hydrophobic polymer is polyethylene, polymethacrylate, polyamide-nylon poly(ethylene-vinyl acetate) cellulose nitrate or a silicone.

5. A varnish composition as claimed in any of claims 1 to 4 wherein the pharmaceutically acceptable solvent comprises ethyl alcohol.

6. A composition as claimed in any of claims 1 to 5 additionally comprising a flavouring agent, a surface active agent, a colouring agent, a plasticizer such as polyethylene glycol, glycerol, sorbitol, or mineral oil, or an adhesive gum, such as gum mastic.

7. The use of a composition comprising:
(a) an anti-hypersensitivity agent; and
(b) a sustained release hydrophobic polymer dissolved in a pharmaceutically acceptable solvent;
in the preparation of a sustained release dental anti-hypersensitivity varnish composition according to claim 1.

8. The use as claimed in claim 7 wherein the anti-hypersensitivity agent is a strontium salt, potassium, lithium or sodium nitrate, potassium bicarbonate, potassium chloride, hydroxyapatite, fluorapatite, ammonium oxalate, EDTA with fluoride, fluoride, or ammonium glycyrrhizzinate.

9. The use as claimed in claim 7 or 8 wherein the hydrophobic polymer is polyethylene, polymethacrylate, polyamide-nylon, poly(ethylene-vinyl acetate) cellulose nitrate or a silicone.

10. The use as claimed in any of claims 7 to 9 wherein the pharmaceutically acceptable solvent comprises ethanol.

## Claims (Claims for the following Contracting State(s): ES, GR)

1. A process for the preparation of a sustained-release dental anti-hypersensitivity varnish composition comprising:
(a) an anti-hypersensitivity agent; and
(b) a sustained release hydrophobic polymer dissolved in a pharmaceutically acceptable solvent;
the composition being adapted to dry to form a film on evaporation of the solvent, which film adheres to a surface of a tooth in a manner which resists removal under normal conditions such as eating or brushing of teeth, and which allows sustained release of the hypersensitivity agent so that a hypersensitive condition can be treated;
the process comprising admixing:
(a) an anti-hypersensitivity agent; and
(b) a sustained release hydrophobic polymer;
and a pharmaceutically acceptable evaporable solvent to dissolve the polymer.

2. A process as claimed in claim 1 wherein the anti-hypersensitivity agent is a strontium salt, potassium, lithium or sodium nitrate, potassium bicarbonate, potassium chloride, hydroxyapatite, fluorapatite, ammonium oxalate, EDTA with fluoride, fluoride, or ammonium glycyrrhizzinate.

3. A process as claimed in claim 1 or 2 wherein the hydrophobic polymer is polyethylene, polymethacrylate, polyamide-nylon, poly(ethylene-vinyl acetate) cellulose nitrate or a silicone.

4. A process as claimed in any of claims 1 to 3 wherein the pharmaceutically acceptable solvent comprises ethyl alcohol.

5. A process as claimed in any of claims 1 to 4 wherein the composition additionally comprises a flavouring agent, a surface active agent, or a colouring agent.

6. A process as claimed in any of claims 1 to 5 wherein the composition additionally comprises a plasticizer such as polyethylene glycol, gycerol, sorbitol, or mineral oil, or an adhesive gum, such as gum mastic.

7. The use of a composition comprising:
(a) an anti-hypersensitivity agent; and
(b) a sustained release hydrophobic polymer dissolved in a pharmaceutically acceptable solvent;
in the preparation of a sustained release dental anti-hypersensitivity varnish composition comprising:
(a) an anti-hypersensitivity agent; and
(b) a sustained release hydrophobic polymer dissolved in a pharmaceutically acceptable solvent;
the sustained release dental hydrophobic varnish composition being adapted to dry to form a film on evaporation of the solvent, which film adheres to a surface of a tooth in a manner which resists removal under normal conditions such as eating or brushing of teeth, and which allows sustained release of the hypersensitivity agent so that a hypersensitive condition can be treated.

8. The use as claimed in claim 7 wherein the anti-hypersensitivity agent is a strontium salt, potassium, lithium or sodium nitrate, potassium bicarbonate, potassium chloride, hydroxyapatite, fluorapatite, ammonium oxalate, EDTA with fluoride, fluoride, or ammonium glycyrrhizzinate.

9. The use as claimed in claim 7 or 8 wherein the hydrophobic polymer is polyethylene, polymethacrylate, polyamide-nylon, poly(ethylene-vinyl acetate) cellulose nitrate or a silicone.

10. The use as claimed in any of claims 7 to 9 wherein the pharmaceutically acceptable solvent comprises ethanol.

## Patentansprüche (Patentansprüche für folgende(n) Vertragsstaat(en): AT, BE, CH, DE, DK, FR, GB, IT, LI, LU, NL, SE)

1. Langzeitwirkende Lackzusammensetzung gegen Zahnüberempfindlichkeit, umfassend:
(a) ein Mittel gegen Überempfindlichkeit und
(b) ein langzeitwirkendes hydrophobes Polymer, das in einem pharmazeutisch verträglichen Lösungsmittel gelöst ist,
wobei die Zusammensetzung so beschaffen ist, daß sie beim Trocknen durch Verdampfen des Lösungsmittels einen Film bildet, welcher an der Zahnoberfläche in einer Weise haftet, die einer Entfernung unter normalen Bedingungen wie Essen oder Bürsten der Zähne widersteht, und die eine Langzeitwirkung des Mittels gegen Überempfindlichkeit gestattet, so daß Überempfindlichkeit behandelt werden kann.

2. Langzeitwirkende Lackzusammensetzung gegen Zahnüberempfindlichkeit, umfassend:
(a) ein Mittel gegen Überempfindlichkeit und
(b) ein langzeitwirkendes hydrophobes Polymer, das in einem pharmazeutisch verträglichen Lösungsmittel gelöst ist,
wobei die Zusammensetzung so beschaffen ist, daß sie beim Trocknen durch Verdampfen des Lösungsmittels einen Film bildet, welcher an der Zahnoberfläche in einer Weise haftet, die einer Entfernung unter normalen Bedingungen wie Essen oder Bürsten der Zähne widersteht, und die eine Langzeitwirkung des Mittels gegen Überempfindlichkeit gestattet, so daß Überempfindlichkeit behandelt werden kann, zur Verwendung in der Medizin.

3. Zusammensetzung nach Anspruch 1 oder 2, wobei das Mittel gegen Überempfindlichkeit ein Strontiumsalz, Kalium-, Lithium- oder Natriumnitrat, Kaliumbicarbonat, Kaliumchlorid, Hydroxylapatit, Fluorapatit, Ammoniumoxalat, EDTA mit Fluorid, Fluorid oder Ammoniumglycyrrhizinat ist.

4. Zusammensetzung nach einem der Ansprüche 1 bis 3, wobei das hydrophobe Polymer Polyethylen, Polymethacrylat, Polyamid-Nylon, Poly(ethylenvinylacetat)cellulosenitrat oder ein Silikon ist.

5. Lackzusammensetzung nach einem der Ansprüche 1 bis 4, wobei das pharmazeutisch verträgliche Lösungsmittel Ethylalkohol umfaßt.

6. Zusammensetzung nach einem der Ansprüche 1 bis 5, die zusätzlich einen Aromastoff, ein Tensid, ein Farbmittel, einen Weichmacher, wie Polyethylenglykol, Glycerin, Sorbit oder Mineralöl, oder ein klebendes Gummi, wie Mastix, umfaßt.

7. Verwendung einer Zusammensetzung, umfassend:
(a) ein Mittel gegen Überempfindlichkeit und
(b) ein langzeitwirkendes hydrophobes Polymer, das in einem pharmazeutisch verträglichen Lösungsmittel gelöst ist,
bei der Herstellung einer langzeitwirkenden Lackzusammensetzung gegen Zahnüberempfindlichkeit nach Anspruch 1.

8. Verwendung nach Anspruch 7, wobei das Mittel gegen Überempfindlichkeit ein Strontiumsalz, Kalium-, Lithium- oder Natriumnitrat, Kaliumbicarbonat, Kaliumchlorid, Hydroxylapatit, Fluorapatit, Ammoniumoxalat, EDTA mit Fluorid, Fluorid oder Ammoniumglycyrrhizinat ist.

9. Verwendung nach Anspruch 7 oder 8, wobei das hydrophobe Polymer Polyethylen, Polymethacrylat, Polyamid-Nylon, Poly(ethylenvinylacetat)cellulosenitrat oder ein Silikon ist.

10. Verwendung nach einem der Ansprüche 7 bis 9, wobei das pharmazeutisch verträgliche Lösungsmittel Ethanol umfaßt.

## Patentansprüche (Patentansprüche für folgende(n) Vertragsstaat(en): ES, GR)

1. Verfahren zur Herstellung einer langzeitwirkenden Lackzusammensetzung gegen Zahnüberempfindlichkeit, umfassend:
(a) ein Mittel gegen Überempfindlichkeit und
(b) ein langzeitwirkendes hydrophobes Polymer, das in einem pharmazeutisch verträglichen Lösungsmittel gelöst ist,
wobei die Zusammensetzung so beschaffen ist, daß sie beim Trocknen durch Verdampfen des Lösungsmittels einen Film bildet, welcher an der Zahnoberfläche in einer Weise haftet, die einer Entfernung unter normalen Bedingungen wie Essen oder Bürsten der Zähne widersteht, und die eine Langzeitwirkung des Mittels gegen Überempfindlichkeit gestattet, so daß Überempfindlichkeit behandelt werden kann,
wobei das Verfahren Mischen von:
(a) einem Mittel gegen Überempfindlichkeit und
(b) einem langzeitwirkenden hydrophoben Polymer und einem pharmazeutisch verträglichen verdampfbaren Lösungsmittel zum Lösen des Polymers umfaßt.

2. Verfahren nach Anspruch 1, wobei das Mittel gegen Überempfindlichkeit ein Strontiumsalz, Kalium-, Lithium- oder Natriumnitrat, Kaliumbicarbonat, Kaliumchlorid, Hydroxylapatit, Fluorapatit, Ammoniumoxalat, EDTA mit Fluorid, Fluorid oder Ammoniumglycyrrhizinat ist.

3. Verfahren nach Anspruch 1 oder 2, wobei das hydrophobe Polymer Polyethylen, Polymethacrylat, Polyamid-Nylon, Poly(ethylenvinylacetat)cellulosenitrat oder ein Silikon ist.

4. Verfahren nach einem der Ansprüche 1 bis 3, wobei das pharmazeutisch verträgliche Lösungsmittel Ethylalkohol umfaßt.

5. Verfahren nach einem der Ansprüche 1 bis 4, wobei die Zusammensetzung zusätzlich einen Aromastoff, ein Tensid oder ein Farbmittel umfaßt.

6. Verfahren nach einem der Ansprüche 1 bis 5, wobei die Zusammensetzung zusätzlich einen Weichmacher, wie Polyethylenglykol, Glycerin, Sorbit oder Mineralöl, oder ein klebendes Gummi, wie Mastix, umfaßt.

7. Verwendung einer Zusammensetzung, umfassend:
(a) ein Mittel gegen Überempfindlichkeit und
(b) ein langzeitwirkendes hydrophobes Polymer, das in einem pharmazeutischen Lösungsmittel gelöst ist,
bei der Herstellung einer langzeitwirkenden Lackzusammensetzung gegen Zahnüberempfindlichkeit, umfassend:
(a) ein Mittel gegen Überempfindlichkeit und
(b) ein langzeitwirkendes hydrophobes Polymer, das in einem pharmazeutisch verträglichen Lösungsmittel gelöst ist,
wobei die langzeitwirkende Lackzusammensetzung gegen Zahnüberempfindlichkeit so beschaffen ist, daß sie bei Trocknen durch Verdampfen des Lösungsmittels einen Film bildet, welcher an der Zahnoberfläche in einer Weise haftet, die einer Entfernung unter normalen Bedingungen wie Essen oder Bürsten der Zähne widersteht, und die eine Langzeitwirkung des Mittels gegen Überempfndlichkeit gestattet, so daß Überempfindlichkeit behandelt werden kann.

8. Verwendung nach Anspruch 7, wobei das Mittel gegen Überempfindlichkeit ein Strontiumsalz, Kalim-, Lithium- oder Natriumnitrat, Kaliumbicarbonat, Kaliumchlorid, Hydroxylapatit, Fluorapatit, Ammoniumoxalat, EDTA mit Fluorid, Fluorid oder Ammoniumglycyrrhizinat ist.

9. Verwendung nach Anspruch 7 oder 8, wobei das hydrophobe Polymer Polyethylen, Polymethacrylat, Polyamid-Nylon, Poly(ethylenvinylacetat)cellulosenitrat oder ein Silikon ist.

10. Verwendung nach einem der Ansprüche 7 bis 9, wobei das pharmazeutisch verträgliche Lösungsmittel Ethanol umfaßt.

## Revendications (Revendications pour l'(les) Etat(s) contractant(s) suivant(s): AT, BE, CH, DE, DK, FR, GB, IT, LI, LU, NL, SE)

1. Composition de vernis anti-hypersensibilité dentaire, à libération entretenue, comprenant :
(a) un agent anti-hypersensibilité ; et
(b) un polymère hydrophobe à libération entretenue, dissous dans un solvant pharmaceutiquement acceptable ;
la composition étant apte à sécher afin de former un film par évaporation du solvant, lequel film adhère à une surface d'une dent de façon à résister à une élimination dans les conditions normales telles que lors de l'absorption d'aliments ou du brossage des dents, et permet la libération entretenue de l'agent anti-hypersensibilité de sorte qu'un état d'hypersensibilité puisse être traité.

2. Composition de vernis anti-hypersensibilité dentaire, à libération entretenue, comprenant :
(a) un agent anti-hypersensibilité ; et
(b) un polymère hydrophobe à libération entretenue, dissous dans un solvant pharmaceutiquement acceptable ;
la composition étant apte à sécher afin de former un film par évaporation du solvant, lequel film adhère à une surface d'une dent de façon à résister à une élimination dans les conditions normales telles que lors de l'absorption d'aliments ou du brossage des dents, et permet la libération entretenue de l'agent anti-hypersensibilité de sorte qu'un état d'hypersensibilité puisse être traité ;
pour utilisation en médecine.

3. Composition selon la revendication 1 ou 2, dans laquelle l'agent anti-hypersensibilité est un sel de strontium, le nitrate de potassium, de lithium ou de sodium, le bicarbonate de potassium, le chlorure de potassium, l'hydroxyapatite, la fluorapatite, l'oxalate d'ammonium, l'EDTA avec un fluorure, un fluorure, ou le glycyrrhizzinate d'ammonium.

4. Composition selon l'une des revendications 1 à 3, dans laquelle le polymère hydrophobe est un polyéthylène, un polyméthacrylate, un polyamide-nylon, un poly(éthylène-acétate de vinyle) nitrate de cellulose ou une silicone.

5. Composition de vernis selon l'une des revendications 1 à 4, dans laquelle le solvant pharmaceutiquement acceptable comprend l'alcool éthylique.

6. Composition selon l'une des revendications 1 à 5, comprenant en outre un agent aromatisant, un agent tensio-actif, un agent colorant, un plastifiant tel que le polyéthylène glycol, le glycérol, le sorbitol, ou une huile minérale, ou une gomme adhésive telle que la gomme-mastic.

7. Utilisation d'une composition comprenant :
(a) un agent anti-hypersensibilité ; et
(b) un polymère hydrophobe à libération entretenue, dissous dans un solvant pharmaceutiquement acceptable ;
dans la préparation d'une composition de vernis anti-hypersensibilité dentaire, à libération entretenue, selon la revendication 1.

8. Utilisation selon la revendication 7, dans laquelle l'agent anti-hypersensibilité est un sel de strontium, le nitrate de potassium, de lithium ou de sodium, le bicarbonate de potassium, le chlorure de potassium, l'hydroxyapatite, la fluorapatite, l'oxalate d'ammonium, l'EDTA avec un fluorure, un fluorure ou le glycyrrhizzinate d'ammonium.

9. Utilisation selon la revendication 7 ou 8, dans laquelle le polymère hydrophobe est un polyéthylène, un polyméthacrylate, un polyamide-nylon, un poly(éthylène-acétate de vinyle) nitrate de cellulose ou une silicone.

10. Utilisation selon l'une des revendications 7 à 9, dans laquelle le solvant pharmaceutiquement acceptable comprend l'éthanol.

## Revendications (Revendications pour l'(les) Etat(s) contractant(s) suivant(s): ES, GR)

1. Procédé de préparation d'une composition anti-hypersensibilité dentaire, à libération entretenue, comprenant :
(a) un agent anti-hypersensibilité ; et
(b) un polymère hydrophobe à libération entretenue, dissous dans un solvant pharmaceutiquement acceptable ;
la composition étant apte à sécher afin de former un film par évaporation du solvant, lequel film adhère à une surface d'une dent de façon à résister à une élimination dans les conditions normales telles que lors de l'absorption d'aliments ou du brossage des dents, et parmet la libération entretenue de l'agent anti-hypersénsibilité de sorte qu'un état d'hypersensibilité puisse être traité ;
le procédé comprenant le mélange :
(a) d'un agent anti-hypersensibilité ; et
(b) d'un polymère hydrophobe à libération entretenue ;
et d'un solvant évaporable pharmaceutiquement acceptable pour dissoudre le polymère.

2. Procédé selon la revendication 1, dans lequel l'agent anti-hypersensibilité est un sel de strontium, le nitrate de potassium, de lithium ou de sodium, le bicarbonate de potassium, le chlorure de potassium, l'hydroxyapatite, la fluorapatite, l'oxalate d'ammonium, l'EDTA avec un fluorure, un fluorure, ou le glycyrrhizzinate d'ammonium.

3. Procédé selon la revendication 1 ou 2, dans lequel le polymère hydrophobe est un polyéthyléne, un polyméthacrylate, un polyamide-nylon, un poly(éthylène-acétate de vinyle) nitrate de cellulose ou une silicone.

4. Procédé selon l'une des revendications 1 à 3, dans lequel le solvant pharmaceutiquement acceptable comprend l'alcool éthylique.

5. Procédé selon l'une des revendications 1 à 4, dans lequel la composition comprend en outre un agent aromatisant, un agent tensio-actif ou un agent colorant.

6. Procédé selon l'une des revendications 1 à 5, dans lequel la composition comprend en outre un plastifiant tel que le polyéthyléne glycol, le glycérol, la sorbitol, ou une huile minérale, ou une gomme adhésive telle que la gomme-mastic,

7. Utilisation d'une composition comprenant :
(a) un agent anti-hypersensibilité ; et
(b) un polymère hydrophobe à libération entretenue, dissous dans un solvant pharmaceutiquement acceptable ;
dans la préparation d'une composition de vernis anti-hypersensibilité dentaire, à libération entretenue, comprenant :
(a) un agent anti-hypersensibilité ; et
(b) un polymère hydrophobe à libération entretenue, dissous dans un solvant pharmaceutiquement acceptable ;
la composition de vernis hydrophobe dentaire à libération entretenue étant apte à sécher afin de former un film par évaporation du solvant, lequel film adhère à une surface d'une dent de façon à résister à une élimination dans les conditions normales telles que lors de l'absorption d'aliments ou du brossage des dents, et permet la libération entretenue de l'agent anti-hypersensibilité de sorte qu'un état d'hypersensibilité puisse être traité.

8. Utilisation selon la revendication 7, dans laquelle l'agent anti-hypersensibilité est un sel de strontium, le nitrate de potassium, de lithium ou de sodium, le bicarbonate de potassium, le chlorure de potassium, l'hydroxyapatite, la fluorapatite, l'oxalate d'ammonium, l'EDTA avec un fluorure, un fluorure ou le glycyrrhizzinate d'ammonium.

9. Utilisation selon la revendication 7 ou 8, dans laquelle le polymère hydrophobe est un polyéthyléne, un polyméthacrylate, un polyamide-nylon, un poly(éthyléne-acétate de vinyle) nitrate de cellulose ou une silicone.

10. Utilisation selon l'une des revendications 7 à 9, dans laquelle le solvant pharmaceutiquement acceptable comprend l'éthanol.
